# EUROPEAN PATENT APPLICATION

(11) **EP 4 477 141 A1**
(43) Date of publication of application: **18.12.2024**
(21) Application number: 23179171.6
(22) Date of filing: 14.06.2023
(51) Int. Cl.: A61B 5/022, A61B 5/00

(54) **SECUREMENT FOR A BLOOD PRESSURE MEASUREMENT CUFF**

(71) Applicant: Koninklijke Philips N.V., 5656 AG Eindhoven (NL)
(72) Inventor: MENA BENITO, Maria Estrella, 5656 AG Eindhoven (NL); SCHMITT, Lars, 5656 AG Eindhoven (NL); PELSSERS, Eduard Gerard Marie, 5656 AG Eindhoven (NL); HILGERS, Achim Rudolf, 5656 AG Eindhoven (NL); WIJSHOFF, Ralph Wilhelm Christianus Gemma Rosa, 5656 AG Eindhoven (NL); DAVIDOIU, Valentina-Geta, 5656 AG Eindhoven (NL)
(74) Representative: Philips Intellectual Property & Standards

(57) **Abstract**

An improved means for securing or fastening a blood pressure measurement cuff by means of a locking mechanism which comprises at least a first and second coupling element which engage through a physical interaction, and wherein the physical interaction is such as to define a discrete set of relative positionings of the first and second coupling element when the fastening is secured.

## Description

### FIELD OF THE INVENTION

The present invention relates to a cuff for use in obtaining hemodynamic parameter measurements.

### BACKGROUND OF THE INVENTION

Blood pressure is among the most measured physiological parameters. It can be used for the assessment of hemodynamic stability. Arterial blood pressure (BP) is the pressure exerted by circulating blood on the arterial vessel walls. It is typically characterized by a set of values: systolic blood pressure (the maximal BP during a given heart cycle), diastolic blood pressure (the minimal BP during a heart cycle), and mean arterial blood pressure (average BP during a heart cycle). In an operating room environment and in an intensive care unit, blood pressure measurements are usually obtained intermittently and non-invasively using oscillometry (upper-arm cuff method) or obtained continuously and invasively with an arterial catheter.

With regards to cuff-based methods, the cuff is attached around a limb (most commonly the upper arm). Once the cuff is wrapped around the limb, the cuff is secured in place by a coupling between the overlapping faces of the cuff.

A common measurement method applied by state of the art oscillometric devices is to conduct the measurement during continuous inflation (or deflation) of the cuff.

For securement of the cuff, state of the art devices use hook-and-loop type couplings (Velcro^{®}) due to its ease of attachment and detachment.

However, during the inflation process of the cuff, it may occur that the hook-and-loop coupling slips, i.e. some of the hook-loop interconnections release. This leads to an artefact in the measurement signal known as a cuff crack. Cuff cracks have been shown to have an impact on the accuracy of the blood pressure measurements.

### SUMMARY OF THE INVENTION

An aim of embodiments of the present invention is to provide an improved means for securing the cuff that eliminates the presence of cuff crack artefacts.

The invention is defined by the claims.

According to examples in accordance with an aspect of the invention, there is provided a system for use in blood pressure measurement of a patient, comprising: an inflatable cuff, adapted to be wrapped around a limb of a patient; and a releasable securement means for securing the wrapped cuff around the arm to prevent unwrapping. The securement means permits reversible transition from an open state in which the cuff is not secured, permitting removal of the cuff from the limb, and a locked state in which the cuff is secured against unwrapping. The securement means comprises a locking mechanism comprising a first coupling part comprising at least one first coupling element and a second coupling part comprising at least one second coupling element, the at least first and second coupling elements for co-operatively engaging with one another by physical interaction.

According to one advantageous set of embodiments, the securement means is configured such that said physical interaction locates the first coupling part relative to the second coupling part in one of a discrete set of one or more positions, for example a user-selected one of a discrete set of one or more positions.

In other words, in this set of embodiments, a fastening means is provided whereby the physical interaction between the first and second coupling elements acts to simultaneously: locate the first element relative to the second element; and secure the first element to the second element. This has the benefit of avoiding the possibility of slippage of the coupling because the coupling permits only a limited, finite set of spatial positionings of the first and second coupling elements relative to one another. This helps avoid the problem of cuff cracking which arises due to slippage of the hook-and-loop coupling. Using the securement means according to the present invention, lateral slippage is not possible.

Thus, the disadvantages of the state of the art hook-and-loop type fastenings may be overcome, and a more robust fastening may be provided which does not give rise to cuff crack artefacts.

In some embodiments, the locking mechanism permits coupling the first and second coupling parts in only one defined relative position. In other embodiments, the locking mechanism permits coupling the first and second coupling parts in any one of a set of one or more discrete positions, wherein a user can selectively couple the coupling parts together in any selected one of those positions.

Different options are possible for implementing the securement means.

According to a first set of embodiments, the securement means is a passive securement means, wherein the physical interaction is a static or passive physical interaction. Examples include a permanent magnetic coupling, wherein the first and second coupling elements comprise mutually attractive permanent magnets; and a mechanical coupling such as a popper coupling, or tongue and groove coupling or key lock coupling.

According to a further set of embodiments, the securement means is an active securement means, by which is meant that its locking state (open vs locked) is actively controllable.

For example, according to some embodiments the at least one first coupling element is electronically addressable to change a physical property of the element, wherein the change of the physical property induces or modifies the physical interaction with the at least one second coupling element to provide securement. The physical interaction may be magnetic interaction or mechanical/physical interaction. As will be explained below, a controllable magnetic state can be achieved using addressable electromagnetic elements, and a controllable mechanical state can be achieved using electronically responsive materials, e.g. electroactive materials such as electroactive polymers. It is noted that the option of providing an electronically addressable coupling is not inextricably linked with the feature that the physical interaction locates the first coupling part relative to the second coupling part in a selected one of a discrete set of one or more positions. Each independently provides a contribution to addressing the problem of improving the securement of the cuff. However, the two features do combine in a synergistic way if implemented together, since the ability to electronically lock and unlock the securement enables simpler implementation of discrete locking positioning of the securement means.

In some embodiments, the system further comprises a controller arranged to control electronic addressing of the at least one first element. The controller may comprise a microprocessor or may comprise control circuitry. All or part of the controller may in some embodiments be integrated into the cuff. In this case, the cuff may include a local power source, for example a battery for powering the at least part of the controller which is integrated in the cuff. In some embodiments, all or part of the controller may be separate from the cuff, for instance integrated in a control module which supplies pressurized air to the cuff.

In some embodiments, the physical property is a magnetic field, and wherein the at least one first coupling element comprises an electromagnet.

Additionally or alternatively, the physical property is a physical shape, and wherein the at least one first coupling element comprises an electroactive material actuable to transition the at least one first coupling elements from a first shape state to a second shape state, for transitioning the securement means from the open state to the locked state.

The electroactive material may be an electroactive polymer.

By way of example, in some embodiments, in the second shape state, the at least one first and second coupling elements physically engage with one another to provide securement and wherein, in the first shape state, the coupling elements are released from one another, to allow for release of the cuff.

In some embodiments, the securement means comprises an assembly of first and second coupling elements, and wherein the elements are arranged to define, in the locked state, an interlock geometry with one another.

By way of example, in some embodiments, the first coupling part comprises a first array of coupling elements and the second coupling part comprises a second array of coupling elements, and wherein the elements of the first array are arranged to interlock with elements of the second array in the locked state.

For example, in the locked state, the first and second array of elements may engage in an interdigitated formation, i.e. may be interdigitally engaged.

For example, the assembly of coupling elements may take the form of a first and second array of protruding members which are arranged to form the interlock geometry in the locked state.

For example, the first array of coupling elements may comprise a first array of ridge members, and the second array of coupling elements may comprise a second array of ridge members and wherein the first and second arrays of ridge members engage to form the interlock geometry, i.e. so as to knit together.

In some embodiments, the transition from the first shape state to the second state shape comprises expansion of a size or position of the coupling elements so as to provide a frictional securement of the interlocked elements to one another. For example, in the case that the electroactive elements are protruding members, e.g. ridge members, the transition from the first to second shape or position state may comprise an expansion of the cross-sectional width of the member so that, when the first and second arrays of members are interdigitally engaged, the actuation of the members causes them to swell laterally, and thus frictionally lock the members against one another.

In some embodiments, the securement can be enhanced by providing each of the coupling elements with a cross-section which has a wedge geometry, i.e. a width-wise cross section which tapers along the length direction of the elements.

It is noted that, although the interlocking arrangement is described in association with use of electroactive material elements, this configuration might alternatively be provided as a passive arrangement.

In some embodiments, the first array and second array may be arranged for meeting one another in opposing fashion when the cuff is wrapped around a limb of a patient, e.g. the two arrays are spaced from one another along the cuff by a distance approximately equal to the circumference of the limb.

In some embodiments, the system can be enhanced by providing a closure sensor comprising one or more sensitive elements arranged to detect contact or proximity between the at least first and second coupling parts. In this case, said electronic addressing of the at least first coupling element may be responsive to the detection of contact or proximity by the one or more sensitive elements. Hence here the device includes touch sensors to detect the closing action of the lines together. This may be provided by a simple pair of electrodes, contact between which is electronically detectable.

In some embodiments, the securement means further comprises a flexible sheet article arranged to removably wrap around at least a portion of the cuff carrying the locking mechanism along an axial dimension of the cuff for providing supplementary securement of the locking mechanism. This may be arranged to apply a pressure between the first and second coupling parts, for example in a radial direction when the cuff is being worn.

Another aspect of the invention is a system for use in blood pressure measurement of a patient, comprising: an inflatable cuff, adapted to be wrapped around a limb of a patient; a releasable securement means for securing the wrapped cuff around the arm to prevent unwrapping; wherein the securement means permits reversible transition from an open state in which the cuff is not secured, permitting removal of the cuff from the limb, and a locked state in which the cuff is secured against unwrapping; wherein the securement means comprises a locking mechanism comprising a first coupling part comprising at least one first coupling element and a second coupling part comprising at least one second coupling element, the at least first and second coupling elements for co-operatively engaging with one another by physical interaction, and wherein the at least one first coupling element is electronically addressable to change a physical property of the element, wherein the change of the physical property induces the physical interaction with the at least one second coupling element to provide securement.

This may be optionally combined with any of the features, options or embodiments outlined above or below in relation to any other aspect of the invention, including the features of any of the dependent claims, with or without the features of claim 1.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates the effect of cuff crack artefacts on a sampled pressure signal;
Fig. 2 schematically illustrates an example securement means;
Fig. 3 is a block diagram of components of an example system in accordance with one or more embodiments;
Fig. 4 illustrates a securement means in accordance with one or more embodiments;
Fig. 5 illustrates a closer view of the securement means of Fig. 4;
Fig. 6 schematically illustrates electroactive expansion of an interlocking coupling element which comprises an electroactive material;
Fig. 7 illustrates a variation on the securement means of Fig. 4 and Fig. 5, in which a contact sensor is implemented;
Fig. 8 schematically illustrates placement of a securement means relative to a cuff;
Fig. 9 schematically illustrates a further optional feature for providing supplementary securement; and
Fig. 10 and Fig. 11 illustrate use of electroactive materials to implement actuable shape-change behavior.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an improved means for securing or fastening a blood pressure measurement cuff by means of a locking mechanism which comprises at least a first and second coupling element which engage through a physical interaction. In some embodiments, the physical interaction is such as to define a discrete set of relative positionings of the first and second coupling element when the fastening is secured. Additionally or alternatively, in some embodiments, wherein the at least one first coupling element is electronically addressable to change a physical property of the element, wherein the change of the physical property induces the physical interaction with the at least one second coupling element to provide securement.

The most common fastening used to secure non-invasive blood pressure (NIBP) cuffs is a hook-and-loop fastener (e.g. Velcro^{®}).

However, during the process of inflating the cuff, the hook and loop fastening can slip due to some of the hook-loop connections breaking. This can induce a type of artefact in the measurement signal known as a cuff crack. Cuff cracks have been shown to have an impact on the accuracy of the blood pressure measurements.

It is proposed in accordance with embodiments of the present invention to provide an improved closing mechanism for the cuff which is more robust against slippage.

For example, according to at least one set of embodiments, a closing configuration or assembly is provided which includes a blocking mechanism which may include an assembly of interlocking protruding members, e.g. interlocking ridges or lines. In some embodiments, the interlocking elements may incorporate an electro-active material, e.g. electroactive polymer (EAP) so as to render them actively controllable.

Embodiments of the present invention reduce occurrence of cuff cracks, which contributes to improvement in the reliability and accuracy of obtained blood pressure measurements.

With regards to the standard hook and loop (Velcro^{®}) fastening means, this type of fastening has the advantage that it can be overextended across the major surface of the arm circumference, evoking a higher radial pressure to maintain the hook-and-loop elements of the Velcro interconnected. However, during the inflation process, the oscillation air pressure signal may be distorted due to Velcro cracks (slippage) that occur as the cuff is pressurized.

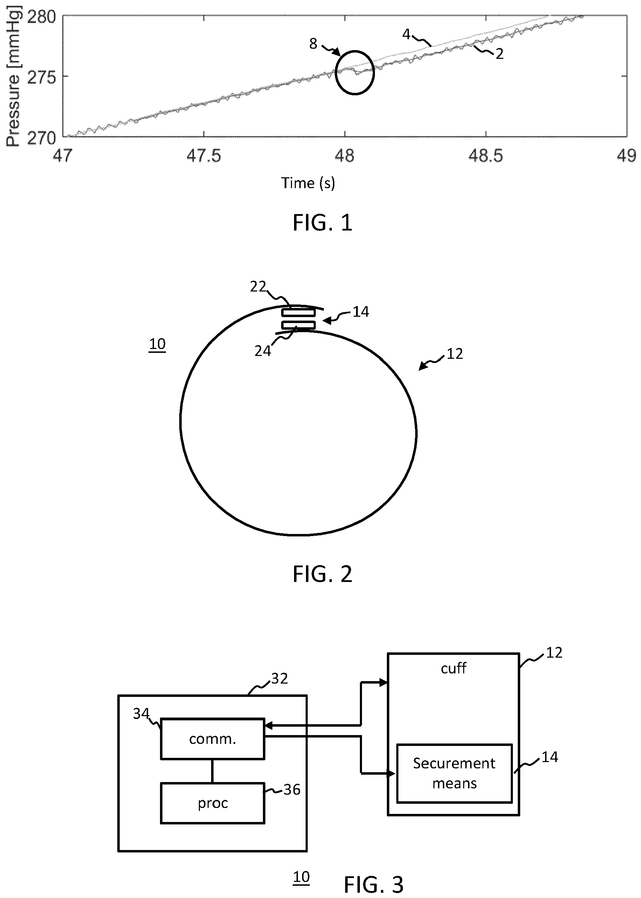

The presence and impact of Velcro' cracks on blood pressure measurements has been investigated by the applicant in a series of experimental tests. Fig. 1 illustrates just one representative example of the impact of a hook-and-loop fastening crack during inflation on the measured pressure signal. The illustrated graph shows a measured pressure signal (y-axis) as a function of time (x-axis) over the course of an inflation of a cuff. This was obtained in a laboratory setup in which a standard NIBP cuff was pressurized when fitted to a rigid phantom arm.

A slip of the fastening (cuff crack) occurs at about time t=48s, and the effect of the cuff crack can be seen in the circled section 8 of the signal. It can be seen that the pressure signal undergoes a sudden negative step change, caused by the sudden slight loosening of the cuff as the fastening slips.

Line 2 in the graph shows the measured pressure signal fluctuating about a baseline. The baseline is computed by removing pump noise from the fluctuating signal. For comparison, line 4 represents an estimate of the pressure signal as would be recorded in the absence of the cuff crack.

The effect of the Velcro' crack on blood pressure measurement precision can be explained as follows. While inflating a cuff, the majority of the hook and loop interconnections of the fasting remain attached, but some may abruptly open, in particular when the cuff is under higher pressure. At this point, a Velcro crack artefact is produced. The sudden opening (Velcro crack) causes the air-volume in the cuff to suddenly increase. This results in an abrupt drop in the air pressure. As shown in Fig. 1, this drop manifests as a reduction in signal amplitude superimposed on the inflation ramp in the pressure signal. In a derivative of the pressure signal, the artefact manifests as a negative impulse.

In addition to negatively impacting blood pressure measurements, any heart rate information derived from the cuff signal would also be impacted. The sudden pressure drop in the signal would modify the oscillating pattern caused by the heartbeat.

Fig. 2 schematically illustrates an example system 10 according to one or more embodiments.

The system 10 is for use in blood pressure measurement of a patient. The system 10 comprises an inflatable cuff 12, adapted to be wrapped around a limb of a patient.

The system further comprises a releasable securement means 14 for securing the wrapped cuff around the arm to prevent unwrapping. The securement means permits reversible transition from an open state in which the cuff is not secured, permitting removal of the cuff from the limb, and a locked state in which the cuff is secured against unwrapping.

The securement means 14 comprises a locking mechanism comprising a first coupling part 22 comprising at least one first coupling element and a second coupling part 24 comprising at least one second coupling element, the at least first and second coupling elements for co-operatively engaging with one another by physical interaction.

The physical interaction may preferably be such as to locate the first coupling part relative to the second coupling part in one of a discrete set of one or more possible positions. In this way, slippage of the fastening, and associated cuff crack artefacts can be avoided.

According to a simplest set of embodiments, the securement means may comprise first and second coupling elements that engage in a purely passive manner and wherein the physical engagement locates the coupling elements of the first and second coupling parts relative to one another in a locked fashion. One example includes the use of a first permanent magnet for the at least one first coupling element and a second permanent magnet for the at least one second coupling element, the first and second permanent magnets being mutually magnetically attractive. A further example includes a mechanical locking mechanism, wherein the first coupling element is arranged to interlock or connect with the second coupling element. This may for example be key lock arrangement or a tongue and groove arrangement or a friction fit fastening arrangement.

In some embodiments, the first coupling part may comprise a plurality of the first coupling elements and the second coupling part may comprise a plurality of the second coupling elements and wherein the first and second coupling parts engage, in the locked state, in an interlocked configuration.

For example, each of the first and second coupling parts may be ridge members, or tooth members, or finger members which engage interdigitally to form an interlock configuration.

According to one particular set of embodiments, the at least first coupling element may be electronically addressable to change a physical property of the element, wherein the change of the physical property induces the physical interaction with the second coupling element to provide securement. In other words the securement means is made actively controllable. In some embodiments, both the first and second coupling elements may be made electronically addressable.

In accordance with this set of embodiments, the system may further comprise a controller arranged to control electronic addressing of the at least first element.

As will be explained in more detail below, in at least one set of embodiments, the physical property is a magnetic field. Here, the at least one first coupling element may comprise an electromagnet. In some embodiments, the first and second coupling elements may each comprise electromagnets. Additionally or alternatively, in at least one set of embodiments, the physical property may be a physical/mechanical shape or size of the at least one first coupling element or a physical position of the coupling element. One way of providing a coupling element having an electronically controllable shape, size or position is to provide the at least one first coupling element comprising an electroactive material, e.g. an electroactive polymer (EAP).

To illustrate this set of embodiments, Fig. 3 schematically illustrates a block diagram of an example system 10 which includes a controller 32 configured to control the electronic addressing of the at least one first and/or at least one second coupling element of the securement means 14, for controlling the locking state of the securement means, i.e. switching the securement means from a locked to unlocked state and vice versa. The system 10 includes the cuff 12 which carries the securement means 14. The controller 32 may comprise one or more processors 36 configured to perform the control function. Alternatively, the controller may include control circuitry to implement the control functionality. In the illustrated example, the controller 32 further comprises an input/output 34 or communication interface for use in communicating a control signal to the securement means 14 and/or for communicative connecting with a pressure sensor of the cuff 12.

All or part of the controller 32 may in some embodiments be integrated into the cuff 12. In this case, the cuff may include a local power source, for example a battery for powering the at least part of the controller which is integrated in the cuff. In some embodiments, all or part of the controller may be separate from the cuff, for instance integrated in a control module which supplies pressurized air to the cuff. The controller may have a local control component integrated in the cuff which is arranged operatively coupled with a master control component located separate to the cuff, for example integrated in a control module which supplies pressurized air to the cuff.

In some embodiments, the system 10 may include a user interface or user control means to enable a user to control locking/unlocking of the securement means 14. In some embodiments, the user interface may be integrated in a control module, wherein the cuff is adapted for fluidic connection to the control module, and wherein the control module includes a pressurized air source for fluidly supplying the cuff.

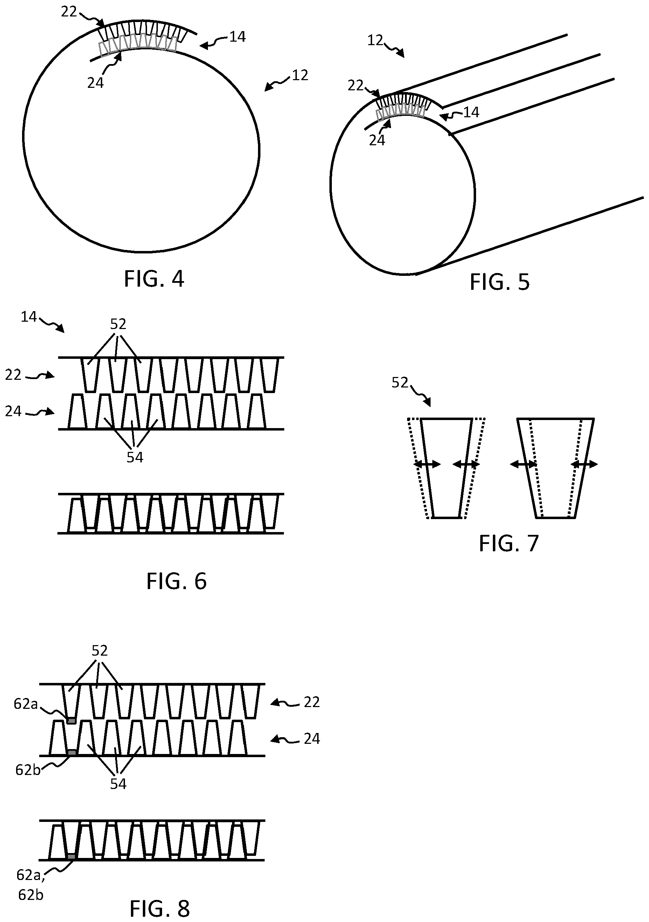

An example securement means in accordance with one or more embodiments is schematically illustrated in Fig. 4, Fig. 5 and Fig. 6.

According to this example, the securement means 14 comprises a first coupling part 22 which comprises a first array of first coupling elements 52 and a second coupling part 24 which comprises a second array of second coupling elements 54, and wherein the elements 52 of the first array are arranged to interlock with elements 54 of the second array in the locked state. The unlocked state is shown in the upper figure of Fig. 5 and the locked state is shown in the lower figure of Fig. 6. As illustrated, the array of first coupling elements 52 engages interdigitally with the array of second coupling elements 54.

As will be recognized, the two interfacing arrays of coupling elements 52, 54 thus provide a locking mechanism, with a first coupling part 22 and second coupling part 24, each with a respective set of coupling elements which co-operatively engage with one another by physical interaction. The physical interaction in this case is mechanical engagement or interlocking. It will further be appreciated that the interlocking engagement of the first and second array of coupling elements acts to locate the first and second coupling parts relative to one another. It will further be appreciated that due to the fact that the coupling is achieved by the interlocking of a discrete set of protruding members 52, 54, this coupling defines only a finite, discrete set of possible securement positions of the first coupling part relative to the second coupling part. In particular, the first coupling part 22 may effectively be incremented in position through each a sequence of possible relative lateral positions relative to the second coupling part 24, each position determined according to the positions of the coupling elements 52, 54.

In the illustrated example, each of the first coupling elements 52 and each of the second coupling elements 54 takes the form of a protruding member, for example a ridge member or a tooth member. In the illustrated example, each of the first and second coupling elements has a wedge geometry, i.e. a width-wise cross-section which tapers along a length of the respective members. Each of the second coupling elements 22 is shaped and configured for being received within a space (i.e. a groove) between two neighboring first coupling elements 24, and vice versa. When the two rows of members slot into one another, they can be said to define an interlock geometry.

As illustrated schematically in Fig. 6, the first coupling elements 52 of the first array 22 are arranged with a regular periodic spacing, and the second coupling elements 54 of the second array 24 are arranged with the same regular periodic spacing.

It will be recognized that due to provision of the first and second arrays of coupling elements, the first and second coupling parts may be secured together in any of a set of discrete positions, each position being defined by engagement of the two arrays at a particular relative lateral position of the two arrays. This allows for securement of the cuff in any of a range of different tightnesses, i.e. different diameters. Furthermore, it will be appreciated that, with this configuration, the securement means is securable in each of a set of different discrete positions, each discrete position defined by a particular lateral positioning of the first coupling part 22 relative to the second coupling part 24.

The pitch of the coupling elements in each array may be made small such that a large number of different securement positions are possible and so that the diameter of the cuff when secured can be finely tuned.

To facilitate this, each of the first and second arrays may be arranged to span in a circumferential direction relative to the cuff when the cuff is positioned on the body part, i.e. to span along a length of the cuff wrap when unfolded. In other words, the coupling elements of each of the first and second arrays may be arrayed along a circumferential dimension of the cuff.

According to at least one set of embodiments, each of the first coupling elements of the first coupling part and each of the second coupling elements of the second coupling part are elongate ridge members, wherein each ridge member has a length which extends along a longitudinal dimension of the cuff when the cuff is worn on the body part. The first and second coupling parts may each comprise an array of the ridge members, wherein the ridge members of each array are distributed along a circumferential dimension of the cuff when the cuff is worn.

Fig. 6 illustrates how, for securing the cuff using the securement means 14, the first coupling part 22 can be pushed against the second coupling part 24 so that the ridge members interlock with one another.

Another way to describe the above-mentioned configuration is that the securement means comprises an assembly of interlocking lines (each line being one of the coupling elements) and wherein, during closure, the lines of the first coupling part 22 and the lines of the second coupling part 24 are pressed gently into one another, whereby displacement or movement of the closing is prevented.

As mentioned above, in one advantageous set of embodiments, the at least one first coupling element 52 of the first coupling part 22 is electronically addressable to change a physical property of the element, wherein the change of the physical property induces the physical interaction with the at least one second coupling element 54 to provide securement.

With regards to the example securement means described above, with reference to Fig. 4, Fig. 5 and Fig. 6, in at least one subset of embodiments, each of at least a subset of the coupling elements 52, 54 may be electronically addressable to change a shape or position of the elements. This change of shape or position may be such as to induce or modify a physical interaction between the coupling elements when in the locked state.

By way of one simple example, one or more actuation means might be provided which are controllable to change a position of at least a subset of the coupling elements to for example strengthen an interlock between the elements. For example, the actuation may cause at least a subset of the elements to press into one another to thereby strengthen an interlock. The actuation means may be mechatronic actuation means, or may be provided by electroactive materials.

According to at least one advantageous set of embodiments, each of at least a subset of the coupling elements 52, 54 may be electronically addressable to change a physical property of the elements, and wherein the physical property is a physical shape, size or position, and wherein the at least subset of the first coupling elements 52 each comprise an electroactive material actuable to transition the respective first coupling element from a first shape, size or position state to a second shape, size or position state, for transitioning the securement means from the open state to the locked state.

By way of example, in accordance with at least one set of embodiments, each of at least a subset of the first coupling elements and/or the second coupling elements may comprise an electroactive material actuable to transition the relevant coupling elements from a first shape and/or size to a second shape and/or size, wherein, in the second shape/size state, the at least subset of coupling elements physically engage with one another and/or other coupling elements to provide locking and wherein, in the first shape/size state, the coupling elements are released from one another, to allow for release of the first and second coupling parts from one another.

An example is illustrated in Fig. 7. In this example, each of at least a subset of the coupling elements 52 comprises an electroactive material, such as an electroactive polymer, and is actuable to transition from a first shape state in which the element has a first, smaller size in at least one dimension, to a second shape state in which the element has a second, larger size in at least one dimension. In the illustrated example of Fig. 7, in the first shape state, the coupling element has a smaller cross-sectional width and, in the second shape state, the element has a larger cross-sectional width. The actuation may be done once the elements of the first and second arrays have been initially interlocked with one another. The actuation has the effect of squeezing neighboring coupling elements against one another laterally, such as to provide a frictional securement of the interlocked elements to one another.

To improve the securement of the interlocked elements to one another, in some embodiments an external surface of the coupling elements may be provided with a non-slip surface. For example, an external surface of each of the coupling elements may be coated or covered with a rubber material, or another non-slip material. In some examples, a non-slip surface may be provided by providing a ridged external surface to the coupling elements for improved interlocking between coupling elements.

Additionally or alternatively to using an electroactive material within the coupling elements, at least a subset of the coupling elements may comprise an electromagnetic component to provide for electronically controllable magnetic attraction between neighboring coupling elements when the elements are interlocked with one another.

In some embodiments, the securement means may take the form of an electroactive hook-and-loop coupling. A standard hook-and-loop fastening comprises a hook side and a loop side. The hook side, also referred to as the "rough" side, comprises a field of tiny, flexible hooks. These are usually made of a stiff material such as nylon or polyester. The hooks are designed to be resilient and flexible to allow for many cycles of connecting and disconnecting. The loop side, also known as the "soft" side, comprises a field of tiny loops. These loops are softer and less rigid than the hooks, allowing the hooks to catch easily. The loops are typically designed to be durable and to withstand repeated cycles of fastening and unfastening.

In accordance with a set of embodiments of the present invention, the second coupling part 24 of the securement means may comprise a standard loop side of a hook-and-loop fastening, while the first coupling part 22 may comprise a hook side comprising a field of multiple hook members, and wherein at least a subset of the hook members comprises an electroactive material and is actuable, by application of an electrical stimulus, to transition from a first shape state to a second shape state. For example, each of the electroactive hook members may be actuable to transition from a less hooked shape to a more hooked shape, i.e. from a more open hook shape to a more closed hook shape. In use, the securement means could be fastened in a two-stage process: in a first stage the first coupling part and second coupling part are simply pressed against one another in the same way as a standard hook-and-loop fastening. In a second stage, a controller may control application of an electrical stimulus to the field of hook members such that the hook members comprising an electroactive material are actuated to deform into a more hooked shape (a more closed hook shape), thereby enhancing the security of the fastening. This would effectively provide an enhanced hook and loop coupling, wherein by application of electrical stimulus to the hook side of the securement means, the securement between the hook and loop sides is strengthened.

A variation on this would be, instead of providing a hook-and-loop fastening, to provide simply the second coupling part comprising an array of loop members, and the first coupling part comprising a corresponding array of hook members, for hooking onto the loop members. At least a subset of the hook members and/or at least a subset of the loop members may be provided comprising an electroactive material and being actuable, by application of an electrical stimulus, to transition from a first shape state to a second shape state. For example, at least a subset of the hook members may be actuable to transition to a more closed hook shape and/or at least a subset of the loop members may be actuable to transition to a smaller diameter loop shape. Initial engagement of the first and second coupling means may be made by hooking the hook members onto the loop members, subsequent to which the hook and/or loop members can be actuated to further secure/lock the securement means.

In accordance with one or more embodiments, the system may further be provided with a closure sensor comprising one or more sensitive elements arranged to detect contact or proximity between the first and second coupling parts. In this way, the electronic addressing of the at least one first coupling element may be made responsive to the detection of contact or proximity by the one or more sensitive elements. In other words, the transition of the securement means from the open to the locked state may be controlled in dependence upon a signal from the closure sensor.

An example is schematically illustrated in Fig 8. Here, a closure sensor is provided which comprises at least one pair of sensitive elements 62a, 62b. This pair of sensitive elements may for example comprise electrodes connected to a detection circuit, wherein contact between the electrodes is detected as completion of the circuit. Any similar configuration may alternatively be employed. In some embodiments, instead of a pair of electrodes, the closure sensor may include an optical proximity sensor disposed on at least one of the coupling elements 52, 54, for detecting proximity between the first and second coupling parts.

Although a single closure sensor is illustrated in Fig. 8, a plurality of sensors may instead be provided. For example each of the coupling elements 52, 54 may be provided with a closure sensor.

In addition to or instead of controlling activation of the locking, the at least one closure sensor may be used for monitoring a closure state of the cuff, and may be used to detect whether there is any slippage or release of the first and second coupling parts during operation. A feedback signal may be generated responsive to such detection for alerting a user for example.

In some embodiments, the system may further comprise a supplementary securement means for adding robustness to the securement of the cuff.

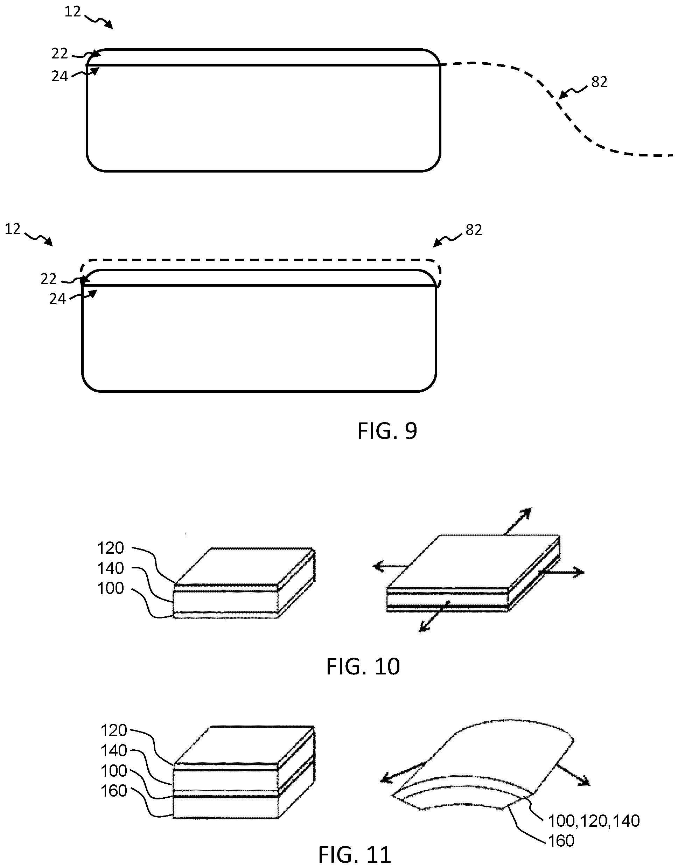

For example, and with reference to Fig. 9, in accordance with one or more embodiments, the securement means further comprises a flexible sheet article 82 arranged to removably wrap around at least a portion of the cuff 12 carrying the locking mechanism along an axial dimension of the cuff for providing supplementary securement of the locking mechanism.

The flexible sheet article is arranged such that, when wrapped around said at least portion of the cuff, it applies a pressure between the first 22, and second coupling 24 parts (i.e. in a radial direction) to improve retention of the two parts to one another.

In some embodiments, the securement means may include a secondary fastening arrangement for further enhancing the security of the securement. For example, in some embodiments in addition to the locking mechanism comprising the first and second coupling parts, the securement means may include a hook-and-loop fastening means. For example, the locking mechanism might span one region of the securement means and the hook-and-loop fastening might span a further region of the securement means. In this way, the locking mechanism effectively provides a block or lock that help prevent the hook-and-loop fastening region from slipping.

As briefly mentioned above, in some embodiments, the use of an electroactive material in the coupling elements may supplemented or replaced with use of magnetic materials in at least a subset of the coupling elements 52, 54.

For example, in one set of embodiments, at least a subset of the first coupling elements 52 of the first coupling part may include a magnet at a distal end of the coupling element, and wherein there may be included a magnetic material in the second coupling part arranged to interface with the magnet(s) of the first coupling part when the two parts are brought together for securement. For example, to take the example embodiment of Fig. 4-6, in some embodiments, a magnet could be included at a distal end of each of the first coupling elements 52, and a magnetic material may be included in the gaps or grooves between each of the second coupling elements 54.

The magnets may be permanent magnets or may be electromagnetic, e.g. electrical coils (with or without a metal core) for generating a magnetic field responsive to application of an electrical current through the coil. In some embodiments, the coils in this case may be printed coils on a (single- or multilayer) flexible printed circuit board (PCB).

Releasing of the two coupling parts 22, 24 could be achieved by either pulling the two ends apart or interrupting the current through the coils.

According to another set of embodiments, magnets may be provided in both the first coupling part 22 and the second coupling part 24, with opposing polarity. This would facilitate a locking between the two parts having greater holding force. For example, this could be implemented by replacing the magnetic material in the example discussed previously with opposite polarity magnets. Again, the magnets may be permanent magnets or electromagnets.

Another aspect of the invention is a system for use in blood pressure measurement of a patient, comprising: an inflatable cuff, adapted to be wrapped around a limb of a patient; a releasable securement means for securing the wrapped cuff around the arm to prevent unwrapping; wherein the securement means permits reversible transition from an open state in which the cuff is not secured, permitting removal of the cuff from the limb, and a locked state in which the cuff is secured against unwrapping; wherein the securement means comprises a locking mechanism comprising a first coupling part comprising at least one first coupling element and a second coupling part comprising at least one second coupling element, the at least first and second coupling elements for co-operatively engaging with one another by physical interaction, and wherein the at least one first coupling element is electronically addressable to change a physical property of the element, wherein the change of the physical property induces the physical interaction with the at least one second coupling element to provide securement.

This may be optionally combined with any of the features, options or embodiments outlined above or below in relation to any other aspect of the invention. By way of just one example, in some embodiments, this aspect of the invention may be implemented with a securement means which takes the form of the electroactive hook-and-loop fastening discussed earlier.

Some embodiments of the invention discussed above employ use of electroactive materials. One class of electroactive material is that of electroactive polymers. Another class of electroactive material is that of piezoelectric materials, where this includes for example certain ceramics such as lead zirconate titanate (PZT), and organic substances such as quartz.

With regards to electroactive polymers (EAPs), these are an emerging class of materials within the field of electrically responsive materials. EAPs can work as sensors or actuators and can easily be manufactured into various shapes allowing easy integration into a large variety of systems. Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications.

An EAP device can be used in any application in which a small amount of movement of a component or feature is desired, based on electric actuation. Similarly, the technology can be used for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Fig. 10 and Fig. 11 show two possible operating modes for an EAP device.

The device comprises an electroactive polymer layer 14 sandwiched between electrodes 10, 12 on opposite sides of the electroactive polymer layer 14.

Fig. 10 shows a device which is not clamped. A voltage is used to cause the electroactive polymer layer to expand in all directions as shown.

Fig. 11 shows a device which is designed so that the expansion arises only in one direction. The device is supported by a carrier layer 16. A voltage is used to cause the electroactive polymer layer to curve or bow.

Together, the electrodes, electroactive polymer layer, and carrier may be considered to constitute the overall electroactive polymer structure.

The nature of this movement for example arises from the interaction between the active layer which expands when actuated, and the passive carrier layer. To obtain the asymmetric curving around an axis as shown, molecular orientation (film stretching) may for example be applied, forcing the movement in one direction.

The expansion in one direction may result from the asymmetry in the EAP polymer, or it may result from asymmetry in the properties of the carrier layer, or a combination of both.

An electroactive polymer structure as described above may be used both for actuation and for sensing. The most prominent sensing mechanisms are based on force measurements and strain detection. Dielectric elastomers, for example, can be easily stretched by an external force. By putting a low voltage on the sensor, the strain can be measured as a function of voltage (the voltage is a function of the area).

Another way of sensing with field driven systems is measuring the capacitance-change directly or measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. O2, NO2), making CNTs usable as gas detectors.

Embodiments of the invention described above employ a controller. The controller may employ control circuitry or may comprise one or more processors. It may be located in a single containing device, structure or unit, or it may be distributed between a plurality of different devices, structures or units. Reference therefore to the controller being adapted or configured to perform a particular step or task may correspond to that step or task being performed by any one or more of a plurality of control components, either alone or in combination. The skilled person will understand how such a distributed control device can be implemented. The controller may include a communication module or input/output for receiving data and outputting data to further components.

If one or more processors are employed, these may be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for use in blood pressure measurement of a patient, comprising:
an inflatable cuff (12), adapted to be wrapped around a limb of a patient;
a releasable securement means (14) for securing the wrapped cuff around the arm to prevent unwrapping;
wherein the securement means permits reversible transition from an open state in which the cuff is not secured, permitting removal of the cuff from the limb, and a locked state in which the cuff is secured against unwrapping;
wherein the securement means comprises a locking mechanism comprising a first coupling part (22) comprising at least one first coupling element (52) and a second coupling part (24) comprising at least one second coupling element (54), the at least first and second coupling elements for co-operatively engaging with one another by physical interaction,
wherein the physical interaction locates the first coupling part relative to the second coupling part in a selected one of a discrete set of one or more positions.

2. The system of claim 1, wherein the at least one first coupling element is electronically addressable to change a physical property of the element, wherein the change of the physical property induces the physical interaction with the at least one second coupling element to provide securement.

3. The system of claim 2, further comprising a controller arranged to control electronic addressing of the at least one first element.

4. The system of claim 2 or 3, wherein the physical property is a magnetic field, and wherein the at least one first coupling element comprises an electromagnet.

5. The system of claim 2 or 3, wherein the physical property is a physical shape, and wherein the at least one first coupling element comprises an electroactive material actuable to transition the at least one first coupling element from a first shape state to a second shape state, for transitioning the securement means from the open state to the locked state.

6. The system of claim 5, wherein, in the second shape state, the at least one first and second coupling elements physically engage with one another to provide securement and wherein, in the first shape state, the coupling elements are released from one another, to allow for release of the cuff.

7. The system of any preceding claim, wherein the securement means comprises an assembly of first and second coupling elements, and wherein the elements are arranged to define, in the locked state, an interlock geometry with one another.

8. The system of claim 7, wherein the first coupling part comprises a first array of first coupling elements and the second coupling part comprises a second array of second coupling elements, and wherein the elements of the first array are arranged to interlock with elements of the second array in the locked state.

9. The system of claim 7 or 8, wherein the assembly of coupling elements takes the form of a first and second array of protruding members which are arranged to form the interlock geometry in the locked state.

10. The system of claim 9, wherein the protruding members are ridge members.

11. The system of claim 5 in combination with any of claims 7-10, wherein the transition from the first shape state to the second state shape comprises expansion of a size of the coupling elements so as to provide a frictional securement of the interlocked elements to one another.

12. The system of any of claims 7-11, wherein a cross section of each of the coupling elements has a wedge geometry.

13. The system of any of claims 2-12, further comprising a closure sensor comprising one or more sensitive elements arranged to detect contact or proximity between the first and second coupling parts, and wherein said electronic addressing of the at least one first coupling element is responsive to the detection of contact or proximity by the one or more sensitive elements.

14. The system of any preceding claim, wherein the securement means further comprises a flexible sheet article arranged to removably wrap around at least a portion of the cuff carrying the locking mechanism along an axial dimension of the cuff for providing supplementary securement of the locking mechanism.

15. A system for use in blood pressure measurement of a patient, comprising:
an inflatable cuff (12), adapted to be wrapped around a limb of a patient;
a releasable securement means (14) for securing the wrapped cuff around the arm to prevent unwrapping;
wherein the securement means permits reversible transition from an open state in which the cuff is not secured, permitting removal of the cuff from the limb, and a locked state in which the cuff is secured against unwrapping;
wherein the securement means comprises a locking mechanism comprising a first coupling part (22) comprising at least one first coupling element (52) and a second coupling part (24) comprising at least one second coupling element (54), the at least first and second coupling elements for co-operatively engaging with one another by physical interaction,
wherein the at least one first coupling element is electronically addressable to change a physical property of the element, wherein the change of the physical property induces the physical interaction with the at least one second coupling element to provide securement.
